(19) Europäisches Patentamt
European Patent Office
Office européen des brevets

(11) **EP 3 338 297 B1**

(12) **EUROPÄISCHE PATENTSCHRIFT**

(45) Veröffentlichungstag und Bekanntmachung des Hinweises auf die Patenterteilung:
**21.08.2019   Patentblatt 2019/34**

(21) Anmeldenummer: **16778191.3**

(22) Anmeldetag: **18.08.2016**

(51) Int Cl.:
*H01J 49/00* *(2006.01)*     *G06K 9/00* *(2006.01)*

(86) Internationale Anmeldenummer:
**PCT/DE2016/000324**

(87) Internationale Veröffentlichungsnummer:
**WO 2017/028838 (23.02.2017 Gazette 2017/08)**

(54) **VERFAHREN ZUR ANALYSE EINES DATENSATZES EINER FLUGZEIT-MASSENSPEKTROMETRIE-MESSUNG UND EINE VORRICHTUNG**

METHOD FOR ANALYSING A DATA SET OF A TIME-OF-FLIGHT MASS SPECTROMETRY MEASUREMENT, AND A DEVICE

PROCÉDÉ D'ANALYSE D'UN BLOC DE DONNÉES D'UNE MESURE PAR SPECTROMÉTRIE DE MASSE DE TEMPS DE VOL, ET DISPOSITIF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.08.2015   DE 102015010602**

(43) Veröffentlichungstag der Anmeldung:
**27.06.2018   Patentblatt 2018/26**

(73) Patentinhaber: **Hochschule Aschaffenburg 63743 Aschaffenburg (DE)**

(72) Erfinder: **LIEB, Floria 63853 Mömlingen (DE)**

(74) Vertreter: **Nitz, Astrid Goethestrasse 23 63739 Aschaffenburg (DE)**

(56) Entgegenhaltungen:
• P. DU ET AL: "Improved peak detection in mass spectrum by incorporating continuous wavelet transform-based pattern matching", BIOINFORMATICS., Bd. 22, Nr. 17, 4. Juli 2006 (2006-07-04), Seiten 2059-2065, XP055259624, GB ISSN: 1367-4803, DOI: 10.1093/bioinformatics/btl355

• VÍCTOR SEGURA ET AL: "Wavelet-based detection of transcriptional activity on a novel Staphylococcus aureus tiling microarray", BMC BIOINFORMATICS, BIOMED CENTRAL, LONDON, GB, Bd. 13, Nr. 1, 5. September 2012 (2012-09-05), Seite 222, XP021138409, ISSN: 1471-2105, DOI: 10.1186/1471-2105-13-222

• DEUKWOO KWON ET AL: "A novel wavelet-based thresholding method for the pre-processing of mass spectrometry data that accounts for heterogeneous noise", PROTEOMICS, Bd. 8, Nr. 15, 1. August 2008 (2008-08-01) , Seiten 3019-3029, XP55313422, DE ISSN: 1615-9853, DOI: 10.1002/pmic.200701010

• P. WANG ET AL: "A dynamic wavelet-based algorithm for pre-processing tandem mass spectrometry data", BIOINFORMATICS., Bd. 26, Nr. 18, 13. Juli 2010 (2010-07-13) , Seiten 2242-2249, XP55313484, GB ISSN: 1367-4803, DOI: 10.1093/bioinformatics/btq403

• N. NGUYEN ET AL: "Mass spectrometry data processing using zero-crossing lines in multi-scale of Gaussian derivative wavelet", BIOINFORMATICS., Bd. 26, Nr. 18, 7. September 2010 (2010-09-07), Seiten i659-i665, XP55313487, GB ISSN: 1367-4803, DOI: 10.1093/bioinformatics/btq397

**Beschreibung**

Die Erfindung betrifft ein Verfahren zur Analyse eines Datensatzes einer Flugzeit-Massenspektrometrie-Messung nach dem Oberbegriff von Anspruch 1 und eine Vorrichtung nach dem Oberbegriff von Anspruch 10.

Ein gemessenes MALDI-Spektrum, Matrix-assisted laser desorption/ionization, wird insbesondere im biologischen Bereich mit einer Vielzahl von biologischen Molekülen bestimmt. Durch eine Beschleunigung von mit einem Laserverfahren abgetragenen, geladenen Teilchen durch ein elektrisches Feld im Rahmen einer Massenspektrometrie-Messung wird ein MALDI-Spektrum erzeugt, wobei Intensitäten von gemessenen Teilchen über einem m/z-Wert bzw. einer Flugzeit aufgetragen werden. Die genaue Bestimmung der Peaks in diesem Spektrum ist für eine Analyse der Zusammensetzung des gemessenen Materials wesentlich und dient beispielsweise der Tumorbestimmung, einer Drogenanalyse oder ähnlichem.

Ein MALDI-Spektrum besitzt eine sogenannte Basislinie, also ein Offset der Intensitäten, der mit zunehmendem Masse-zu-Ladung Verhältnis, m/z-Wert, abnimmt, was vermutlich durch eine ungleichmäßig aufgetragene Matrix von zu messendem Material entsteht.

Weiterhin kann man beobachten, dass die Varianz des Rauschens im Spektrum ebenfalls mit zunehmendem m/z-Wert abnimmt.

Die beiden Effekte können allerdings für jedes Spektrum verschieden sein, wobei ein Datensatz bis zu etwa 100.000 solcher Spektren beinhalten kann.

Somit wird im Stand der Technik eine Vorprozessierung der Datensätze durchgeführt, zum einen eine Filterung bzw. Glättung der Daten, um das Rauschverhalten zu beeinflussen, zum anderen muss die Baseline entfernt werden, um eine sinnvolle Analyse der Peakintensitäten zu gewährleisten.

Ein Beispiel des Standes der Technik ist in Du et al., Bioinformatics, Bd. 22, Nr. 17, 2006, 2059-2065 (DOI: 10.1093/bioinformatics/bti355) gegeben.

Nachteilig dabei ist, dass durch die Filterung und das Entfernen der Baseline auch wichtige Signalanteile wie z.B. Peaks verschwinden oder abgeschwächt werden können. Kleine Peaks, die vielleicht zur Auswertung für den Biologen von Belang gewesen wären, könnten im Verlauf des Preprocessing verschwinden und dann im darauffolgenden Schritt der Peakdetektierung auch nicht mehr erkannt werden.

Aufgabe der vorliegenden Erfindung ist es, eine sichere und einfache Analyse von MALDI -Spektren zu gewährleisten, ohne dass durch eine Vorbearbeitung wichtige Informationen, wie beispielsweise Peaks, verloren gehen.

Die Aufgabe wird gelöst durch ein Verfahren zur Analyse eines Datensatzes einer Flugzeit-Massenspektrometrie-Messung zur Detektion von Intensitätsmaxima in Form von Peaks, wobei eine Flugzeit von beschleunigten Massen mit Ladungen gemessen wird, insbesondere eines Datensatzes umfassend eine Intensität der Ionenhäufigkeit bezogen auf eine Masse pro Ladung, insbesondere von biologischen Molekülen, insbesondere gewonnen aus einem MALDI-Verfahren, Matrix-assisted laser desorption/ionization, wobei Peakinformation von Rauschen getrennt wird,

wobei mittels eines Transformationsmittels eine 2-Parameter-Transformation aus dem Datensatz der Flugzeit-Massenspektrometrie-Messung in einen Zeit-Frequenz-Phasenraum vorgenommen wird, wobei der Zeit-Frequenz-Phasenraum der Raum ist, der von den zwei Parametern (k, l) der 2-Parameter-Transformation aufgespannt wird, wobei mit dem Transformationsmittel eine Wavelet- Transformation oder eine Gabor Transformation vorgenommen wird, wobei mit einem Datensatzaufteilungsmittel das Signal des Datensatzes in einzelne, zu vergleichende Signalabschnitte $f_n$ unterteilt wird, wobei aufeinanderfolgende Signalabschnitte $f_n$ und $f_{n+1}$ insbesondere eine vorbestimmte Überlappung in einem Fenster aufweisen, insbesondere etwa 25%, 30% , 35% oder 50 % einer vorbestimmten Fensterbreite, wobei durch das Transformationsmittel ein Zusammenhang des Signals $f_n$ zu dem zweiten, benachbarten Signal $f_{n+1}$ hergestellt wird dadurch, dass zur Kennzeichnung der Ähnlichkeit der zwei Signale $f_n$ und $f_{n+1}$ ein Multiplier $G_m: L^2(\mathbb{R}) \to L^2(\mathbb{R})$, der Wavelet-Multiplier oder der Gabor-Multiplier, bestimmt wird, der das erste Signal $f_n$ in das zweite $f_{n+1}$ überführt,

$$f_{n+1} = G_m f_n,$$

insbesondere in einer Näherung

$$f_{n+1} = G_m f_n \qquad = V_g^*(m \cdot V_g f_n)$$

wobei

$V_g: L^2(\mathbb{R}) \to \ell^2(\mathbb{Z}^2)$ = Analysisoperator der 2-Parameter-Transformation, der Wavelet-Transformation oder der Gabor Transformation, mit einem Fensterfunktionsmittel g(t),

$V_g^*$ = zugehöriger adjungierter Operator der 2-Parameter-Transformation,

$G_m : L^2(\mathbb{R}) \to L^2(\mathbb{R})$ = Multiplier, Gabor Multiplier oder Wavelet-Multiplier, der die 2-Parameter-Transformation zwischen dem ersten Signals $f_n$ zu einem zweiten Signal $f_{n+1}$ mit unterschiedlichen Phasenraumcharakteristika, insbesondere Zeit-Frequenz(modulieren) Charakteristika durch multiplikative Modifikation beschreibt, wobei

$m \in \ell^\infty(\mathbb{Z}^2)$ = Maske des jeweiligen Multipliers, Wavelet oder Gabor Multipliers,

wobei durch Anpassung einer Maske m eine Näherung durch Multiplikation im Bereich Zeit/Frequenz vorgenommen wird,

wobei die Maske $m$ nahezu 1, entspricht, wenn die Signale $f_n$ und $f_{n+1}$ sehr ähnlich sind,

wobei die Maske m von 1 verschieden ist, wenn in $f_n$ und/oder $f_{n+1}$ ein Peak existiert, der nicht in dem anderen Bereich $f_n$ und/oder $f_{n+1}$ vorkommt.

Durch das erfindungsgemäße Verfahren entfällt eine sehr zeit- und rechenaufwendig Vorbearbeitung der Spektren. Vorteilhaft ist es, dass keine vorverarbeitenden Schritte wie Filterung/Glättung oder Baseline Korrektur notwendig sind, um Peaks zu detektieren.

Der Datensatz wird in Teile $f_n$ unterteilt, wobei aufeinanderfolgende Teile eine vorbestimmte Überlappung aufweisen. Anschließend wird die zugehörige Maske m berechnet, so dass benachbarte Teile $f_n$, $f_{n+1}$ ineinander transformiert werden. Wenn benachbarte Teile unähnlich sind, wird in einem der Teile ein Peak detektiert. Durch den Multiplier $G_m$ wird die Transformation eines Signals $f_n$ mit einer Fensterfunktion $g$, einer anschließenden Gewichtung der Zeit-Frequenz-Koeffizienten mit der Maske $m$ und einer Rücktransformation zu einem Signal $f_{n+1}$ vorgenommen, insbesondere Gabor-Transformation oder Wavelet-Transformation.

Bei der Wavelet-Transformation wird - wie bei der Kuzzeitfouriertransformation - das zu untersuchende Signal mit einer Fensterfunktion verglichen, wobei allerdings das Fenster im Frequenzbereich nicht verschoben und moduliert wird sondern verschoben und skaliert. Durch die Skalierung ergibt sich ebenfalls eine Frequenzverschiebung, allerdings wird gleichzeitig mit einer Frequenzerhöhung die Zeitdauer, d.h. Breite im Zeitbereich des Fensters verringert. Dadurch ergibt sich bei höheren Frequenzen eine bessere zeitliche Auflösung und bei niedrigen Frequenzen eine bessere Frequenzauflösung , wobei die Zeitauflösung schlechter wird.

Enthält nun ein Signal Frequenzanteile sowohl bei hohen als auch bei niedrigen Frequenzen, soll bei niedrigen Frequenzen eine gute Frequenzauflösung erreicht werden, da eine kleine absolute Frequenzänderung hier stark ins Gewicht fällt. Bei einer hohen Frequenz ist eine gute Zeitauflösung wichtiger.

Dadurch, dass nacheinander immer nur ein kleiner Teilabschnitt des ganzen Spektrums mit dem darauffolgenden verglichen wird, ist der Einfluss der Baseline vernachlässigbar klein. Beispielsweise werden bei einem Spektrum mit etwa 7000 Datenpunkten immer nur etwa 100 aufeinanderfolgende Datenpunkte miteinander verglichen, ob in diesen ein Peak vorhanden ist. Somit kann der Einfluss der Baseline in diesem Intervall als konstant angenommen werden, beziehungsweise als eine Funktion die sich in diesem Abschnitt nur leicht linear ändert. Da der Algorithmus aber auf große zeitliche Änderungen des Signals reagiert, wird der Einfluss der Baseline ignoriert. Große zeitliche Änderungen bedeuten hier, dass diese Änderungen in einem sehr kurzen Zeitraum auftauchen, nämlich immer dann wenn ein Peak vorhanden ist, während die Baseline eine viel größere zeitliche Ausdehnung besitzt. Diese große zeitliche Ausdehnung wird vom Algorithmus ignoriert.

Generell besitzen Peaks in MALDI-Spektren ein spezifisches Zeit-Frequenzverhalten, dies bedeutet das Frequenzintervall in dem k läuft, kann genauer spezifiziert werden. Sei z.B. das MALDI-Signal mit einer Abtastrate von 10kHz aufgenommen worden, so würde eine normale Zeit-Frequenzdarstellung alle Frequenzen von 0 bis zur halben Abtastrate 5kHz darstellen. Sind die wichtigen MALDI-Peaks aber nur im Frequenzband von 500 bis 2000Hz, so kann der Analyseoperator $V_g$ dementsprechend angepasst werden nur dieses Frequenzband darzustellen.

Es besteht weiterhin die Möglichkeit auf Peak-Formen einzugehen, die für kleine Massen asymmetrisch sind. Eine Änderung der Frame Parameter, z.B. mit einem asymmetrischen Fenster, wie mit einer vorbestimmten Gauß-Funktion, in einem vorbestimmten m/z Bereich, kann die Peakdetektierung noch verfeinert werden und auf bestimmte Peak-Formen eingegangen werden.

Weiterhin wird durch das erfindungsgemäße Verfahren das sich ändernde Rauschverhältnis berücksichtigt. In den MALDI-Rohdaten ist zu beobachten, dass das Rauschen mit zunehmendem m/z-Wert geringer wird. Durch die Aufteilung des Gesamtsignals in kleinere, sich überlappende Teilstücke wird auf die Varianz des Rauschens besser eingegangen.

Die Sensitivität des Algorithmus' wird im Wesentlichen durch wenige Parameter, insbesondere den λ - Parameter beschrieben. Da dieser für jedes Teilstück separat anhand des in diesem Teilstück auftretenden Rauschens bestimmt werden kann, kann mit einem größeren λ am Anfang des Signals der großen Varianz des Rauschens entgegengewirkt werden. Mit zunehmenden m/z Wert wird dieser Parameter nun sukzessive verringert. Dies hat zur Folge, dass bei größeren m/z Werten die Sensitivität des Algorithmus ebenfalls größer ist, und somit auch kleine Peaks detektiert werden können.

Weiterhin sind nur wenige Parameter für eine Feinanalyse notwendig. Der Algorithmus besitzt nur einen einzigen Parameter, nämlich λ, der wesentlichen Einfluss auf die Peakdetektierung nimmt. Die restlichen Parameter des Algorithmus beeinflussen das Ergebnis der Peakdetektierung nur in sehr geringem Ausmaß. Beispiel: Wählt man anstelle von 50% Überlapp der einzelnen Teilstücke des Signals nur 25%, 30% oder 35%, so zeigt sich, dass immer die gleichen Peaks detektiert werden, unabhängig vom verwendeten Überlapp. Dasselbe gilt für die Länge der einzelnen Teilstücke oder die Frame Parameter. Dies macht die Handhabung äußert komfortabel und sorgt dafür dass die Analyse stabil und zuverlässig sehr stabil arbeitet.

Vorteilhaft ist es, wenn für die Fensterfunktion g(t) gilt für die Gabor-Transformation $V_g$ angewandt auf ein Signal *f(t)* gilt:

$$V_g f\,(x,\omega) = \int_{\mathbb{R}} f(t)\,\bar{g}(t-x)e^{-2\pi i t\omega}dt$$

und für die Wavelet-Transformation $V_g$ angewandt auf ein Signal *f(t)*:

$$V_g f\,(x,a) = a^{-1/2}\int_{\mathbb{R}} f(t)\,\bar{g}(a^{-1}\cdot(t-x))dt$$

Vorteilhaft ist es, wenn der Multiplier $G_m$, insbesondere der Wavelet-Multiplier und/oder der Gabor-Multiplier, in einer linearen Näherung nach der folgenden Gleichung bestimmt wird:

$$m = \arg\min_m \| f_{n+1} - G_m f_n \|^2 + \lambda d(m)$$
$$= \arg\min_m \left\| V_g^*\big(V_g f_{n+1} - m\cdot V_g f_n\big) \right\|^2 + \lambda d(m)$$

wobei

λ = Regularisierungsparameter,
d(m) = ein Regularisierungsterm ist, wobei der Regularisierungsterm so gewählt wird, dass er möglichst klein ist, wenn sich die beiden Signale $f_n$ und $f_{n+1}$ ähnlich sind, insbesondere in beiden Signalen ähnliche Peaks vorhanden sind.

Durch den Parameter λ wird die Empfindlichkeit des Transformations- und Analysevorgangs erreicht. Die Minimierung besteht aus einer Summe von 2 Summanden. Der erste Summand vergleicht - für ein festes m -, wie ähnlich die Signale $f_n$ und $f_{n+1}$ sind, indem ein Wert $\| f_{n+1} - G_m f_n \|^2 = \left\| V_g^*\big(V_g f_{n+1} - m\cdot V_g f_n\big) \right\|^2$ zurückgegeben wird. Ist dieser Wert klein, sind $G_m f_n$ und $f_{n+1}$ ähnlich- für ein festes m -. Ist dieser Wert groß, unterscheiden sich $G_m f_n$ und $f_{n+1}$.

Der zweite Term legt eine Eigenschaft der Maske m fest, die mit dem Parameter λ gewichtet wird. Wird λ sehr groß gewählt, dann wird die Summe minimiert, indem d(m) möglichst klein gewählt wird, denn dann übertrifft der hintere Term den vorderen in der Summe, was bedeutet, für große λ kann man große Unterschiede in $f_n$ und $f_{n+1}$ ausblenden, z.B. starkes Rauschen.

Mit kleiner werdendem λ wird der Einfluss des ersten Terms immer größer. Die größten Unterschiede zwischen $f_n$ und $f_{n+1}$ werden jetzt nicht mehr unterdrückt, sondern müssen von der Maske m ausgeglichen werden. Mit größten Unterschieden zwischen $f_n$ und $f_{n+1}$ ist dann ein auftretender Peak in einem der beiden Signalteile gemeint.

Je kleiner der Wert λ wird, umso mehr Gewicht legt man auf den ersten Term und umso mehr Unterschiede zwischen $f_n$ und $f_{n+1}$ können detektiert werden.

Wählt man λ = 0, lässt man jeden Unterschied zwischen $f_n$ und $f_{n+1}$ zu und somit wird kein Rauschen unterdrückt.

Vorteilhaft ist es, wenn als Regularisierungsterm d(m) = ||m|-1||₁ verwendet wird, wobei eine Abweichung vom absoluten Wert 1 der Maske *m* erzwungen wird und/oder d(m) = ||m- 1||² zur Kontrolle der Energie der Maske verwendet wird.

Vorteilhaft ist es, wenn eine diagonale Näherung verwendet wird, wobei im Zeit-Frequenz Phasenraum angesetzt wird:

$$m = \arg\min_m \sum \sum_{k,l \in \mathbb{Z}} \left| c_{k,l}^{n+1} - m_{k,l} c_{k,l}^n \right|^2 + \lambda d(m).$$

wobei das Signal $f \in L^2(\mathbb{R})$ in diskreter Zerlegung im Zeit-Frequenz Phasenraum dargestellt wird

$$f = V_g^* V_g f = \sum \sum_{k,l \in \mathbb{Z}} c_{k,l} g_{k,l} = \sum \sum_{k,l \in \mathbb{Z}} \langle f, g_{k,l} \rangle g_{k,l}$$

mit $c_{k,\ell} = \langle f, g_{k,\ell} \rangle$ = Zeit-Frequenz Koeffizienten von $f_n$ im Phasenraum (k,l).
wobei insbesondere $g_{k,l} = e^{2\pi i l \omega 0 t} g(t - kx_0)$ = modulierte, translatierte Fensterfunktionen g im Rahmen der Gabor-Transformation,

wobei insbesondere $g_{k,l} = 2^{-\frac{l}{2}} g(2^{-l}(t - kx_0))$ modulierte, translatierte Fensterfunktionen g im Rahmen der Wavelet-Transformation
wobei in Abhängigkeit vom Regulierungsterm d(m) und/oder dem Regulierungsparameter $\lambda$ gelöst wird:

1.

$$d(m) = \|m - 1\|^2$$

$$m_{k,l} = \frac{\left| \overline{c_{k,l}^n} c_{k,l}^{n+1} \right| + \lambda}{\left| c_{k,l}^n \right|^2 + \lambda} \cdot e^{i \arg\left[ \overline{c_{k,l}^n} c_{k,l}^{n+1} \right]}$$

1.

$$d(m) = \||m| - 1\|_1$$

$$m_{k,l} = \begin{cases} \dfrac{\left| \overline{c_{k,l}^n} c_{k,l}^{n+1} \right| - \dfrac{\lambda}{2}}{\left| c_{k,l}^n \right|^2} \cdot e^{i \arg\left[ \overline{c_{k,l}^n} c_{k,l}^{n+1} \right]}, & \text{wenn } \dfrac{\left| \overline{c_{k,l}^n} c_{k,l}^{n+1} \right|}{\left| c_{k,l}^n \right|^2} \geq 1 + \dfrac{\lambda}{2 \left| c_{k,l}^n \right|^2} \\[4ex] \dfrac{\left| \overline{c_{k,l}^n} c_{k,l}^{n+1} \right| + \dfrac{\lambda}{2}}{\left| c_{k,l}^n \right|^2} \cdot e^{i \arg\left[ \overline{c_{k,l}^n} c_{k,l}^{n+1} \right]}, & \text{wenn } \dfrac{\left| \overline{c_{k,l}^n} c_{k,l}^{n+1} \right|}{\left| c_{k,l}^n \right|^2} \leq 1 - \dfrac{\lambda}{2 \left| c_{k,l}^n \right|^2} \\[4ex] \equiv 1, & \text{sonst} \end{cases}$$

wobei hierdurch wiederum deutlich wird, dass je kleiner Parameter $\lambda$ desto näher liegen die Ergebnisse an den gesuchten Peaks.

Vorteilhaft ist es, wenn $m_{k,l}$ die Maske, $k$ der Zeitindex und $l$ der Frequenzindex so wird die exakte Zeitposition $f_k$ eines Peaks derart bestimmt, dass für jeden Zeitpunkt die Absolutbeträge der Frequenzen aufsummiert werden: $f_k = \sum_l |m_{k,l}|$.
Vorteilhaft ist es, wenn es in Verbindung mit einem bildgebenden Verfahrens, insbesondere zur Darstellung eines Ausgabebildes mit Peakdarstellungen, eingesetzt wird.
Die Aufgabe wird gelöst durch eine Verwendung eines Verfahrens nach einem der Ansprüche 1 bis 7, für die Messung von Proteinen und/oder Peptiden und/oder Oligonukleotiden und/oder Oligosachariden verwendet wird.

Die Aufgabe wird gelöst durch eine Verwendung eines Verfahrens nach einem der Ansprüche 1 bis 7, zur Messung von Medikamenten und/oder Drogen und/oder Dopingmittel und/oder Gewebeschnitten und/oder Tumorausbreitungsproben.

Die Aufgabe wird ebenfalls gelöst durch eine Vorrichtung zur Analyse zur Anwendung eines Verfahrens nach einem der Ansprüche 1 bis 9, wobei vorgesehen ist: ein Mittel zur Erfassung von MALDI-Datensätzen, ein Mittel zum Berechnen eine 2-Parameter-Transformation, insbesondere Wavelet-Transformation und/oder Gabor-Transformation, nach einem der Ansprüche 1 bis 9, ein Mittel zur Ausgabe des Berechnungsergebnisses mit einem Ergebnis, ob ein Peak vorliegt oder nicht.

Hierdurch kann die exakte Position des Peaks mit Hilfe der approximierten Maske genauer bestimmt werden. Ein einzelner Parameter $\lambda$ bestimmt die Empfindlichkeit des Verfahrens. Es gibt noch weitere Parameter, insbesondere Frame Parameter, Länge der einzelnen Teilstücke, Überlapp und weitere, aber kleine Änderungen dieser Parameter haben auch nur relativ geringe bis gar keine Auswirkung auf die Peakdetektion. Länge und Überlapp zum Beispiel wirken sich hauptsächlich auf die Performance des Verfahrens aus. Die Wahl der Frame Parameter beeinflusst die gefundenen Peaks nur marginal. Daher ist das Verfahren sehr stabil.

Weitere Merkmale und Vorteile der Erfindung ergeben sich aus den Ansprüchen und der nachstehenden Beschreibung, in der Ausführungsbeispiele des Gegenstands der Erfindung in Verbindung mit der Zeichnung näher erläutert sind.

Es zeigt

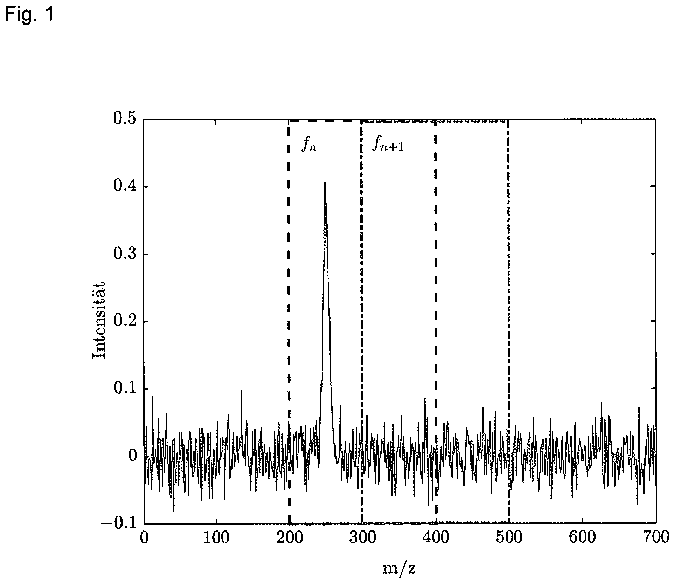

Fig. 1    einen schematischem Datensatz mit Bereich $f_n$ und $f_{n+1}$.

Fig. 1 zeigt einen schematischem Datensatz mit Bereich $f_n$ und $f_{n+1}$, die im erfindungsgemäßen Verfahren verglichen werden.

Der schematische Datensatz wird schematisch in Teile $f_n$ unterteilt, wobei aufeinanderfolgende Teile eine vorbestimmte Überlappung aufweisen. Anschließend wird die zugehörige Maske m berechnet, so dass benachbarte Teile $f_n$, $f_{n+1}$ ineinander transformiert werden. Wenn benachbarte Teile unähnlich sind, wird in einem der Teile ein Peak detektiert. Durch den Multiplier $G_m$ wird die Transformation eines Signals $f_n$ mit einer Fensterfunktion g, einer anschließenden Gewichtung der Zeit-Frequenz-Koeffizienten mit der Maske m und einer Rücktransformation zu einem Signal $f_{n+1}$ vorgenommen, insbesondere Gabor-Transformation oder Wavelet-Transformation.

Beispielhaft wird die Vorgehensweise im Folgenden anhand des Gabor-Transformationsverfahrens in Anwendung auf einen beispielhaften Datensatz gewonnen aus einer Flugzeit-Massenspektrometrie dargestellt, wobei die Eigenschaften eines Signals $f_1$ mit einem anderen Signal $f_2$ verglichen werden, was verallgemeinert $f_n$, $f_{n+1}$ wäre.

Es sei $V_g : L^2(\mathbb{R}) \to \ell^2(\mathbb{Z}^2)$ der diskrete Analyseoperator, auch Gabor Transformation mit Fenster g genannt, sowie $V_g^*$ der zugehörige adjungierte Operator. Ein Signal $f \in L^2(\mathbb{R})$ kann mit Hilfe der Fensterfunktion g in einen (diskreten) Zeit-Frequenz-Raum zerlegt werden und aus diesen Zeit-Frequenz-Koeffizienten $c_{k,l}$ wieder rekonstruiert werden als:

$$f = V_g^* V_g f = \sum_{k,l \in \mathbb{Z}} \sum c_{k,l} g_{k,l} = \sum_{k,l \in \mathbb{Z}} \sum \langle f, g_{k,l} \rangle g_{k,l}$$

Im Rahmen dieser Erfindungsbeschreibung bestehe $g_{k,l}$ aus nur modulierten und translatierten Versionen der Fensterfunktionen $g_{k,l} = e^{2\pi i l \omega_0 t} g(t - kx_0)$. Es ist aber auch möglich hier andere Frames zu benutzen, z.B. eines das aus translatierten und skalierten Fensterfunktionen besteht, oder Frames die ein unregelmäßiges Diskretisierungsschema besitzen (Nonstationary Gabor Frames).

Der Gabor Multiplier $G_m : L^2(\mathbb{R}) \to L^2(\mathbb{R})$ beschreibt die Transition eines bestimmten Signals $f_1$ zu einem anderen Signal $f_2$ mit unterschiedlichen Zeit-Frequenz Charakteristika durch multiplikative Modifikation der Zeit-Frequenz Koeffizienten ($c_{k,l} = \langle f, g_{k,l} \rangle$) von $f_1$. In anderen Worten

$$f_2 = G_m f_1 = V_g^* (m \cdot V_g f_1)$$

wobei $m \in \ell^\infty(\mathbb{Z}^2)$ die sogenannte Maske des Gabor Multipliers ist. Prinzipiell besteht ein Gabor Multiplier also aus der Gabor Transformation eines Signals f mit einer Fensterfunktion g, einer anschließenden Gewichtung der Zeit-Frequenz-Koeffizienten mit der Maske $m$ und einer Rücktransformation.

Somit können diese Multiplier Auskunft darüber geben, ob sich zwei Signale ähnlich sind oder ob sie signifikante Unterschiede im Zeit-Frequenz Verhalten besitzen. Sind die Signale sehr ähnlich um einen bestimmten Punkt in der Zeit-Frequenz Ebene so wird die Maske $m$ um diesen Punkt relativ nahe am multiplikativen Neutralelement, der 1, sein.

Genauso gut kann dieser Gedanke umgekehrt werden: Wir haben zwei Signale $f_1$ und $f_2$ und wir suchen den Gabor Multiplier der das erste Signal in das zweite überführt. Somit kann folgendes inverses Problem definiert werden:

$$m = \arg\min_m \| f_2 - G_m f_1 \|^2 + \lambda d(m)$$

$$= \arg\min_m \left\| V_g^* \left( V_g f_2 - m \cdot V_g f_1 \right) \right\|^2 + \lambda d(m)$$

wobei $d(m)$ ein Regularisierungsterm und $\lambda$ der Regularisierungsparameter ist. Der Regularisierungsterm wird so gewählt, dass dieser möglichst klein ist, wenn sich die beiden Signale $f_1$ und $f_2$ "ähnlich" sind. Im Folgenden wird $d(m) = \|m\| - 1\|_1$ verwendet, wenn die Sparsity der Abweichung vom absoluten Wert 1 der Maske m erzwungen werden soll. Weiterhin kann aber auch $d(m) = \|m - 1\|^2$ zur Kontrolle der Energie der Maske verwendet werden.

Da obiges Minimierungsproblem nicht ganz so einfach zu lösen ist und eine "diagonale" Annäherung in den Experimenten gute Ergebnisse erzielt hat, wird eben diese Annäherung verwendet. Dies bedeutet, dass das Minimierungsproblem direkt in der Zeit-Frequenz Domäne formuliert wird:

$$m = \arg\min_m \sum \sum_{k,l \in \mathbb{Z}} \left| c_{k,l}^2 - m_{k,l} c_{k,l}^1 \right|^2 + \lambda d(m).$$

Je nach Regularisierungsterm hat das vereinfachte Problem in obiger Gleichung folgende explizite Lösung:

$$d(m) = \|m - 1\|^2$$

$$m_{k,l} = \frac{\left| \overline{c_{k,l}^1} c_{k,l}^2 \right| + \lambda}{\left| c_{k,l}^1 \right|^2 + \lambda} \cdot e^{i \arg\left[ \overline{c_{k,l}^1} c_{k,l}^2 \right]}$$

$$d(m) = \| |m| - 1 \|_1$$

$$m_{k,l} = \begin{cases} \dfrac{\left| \overline{c_{k,l}^1} c_{k,l}^2 \right| - \dfrac{\lambda}{2}}{\left| c_{k,l}^1 \right|^2} \cdot e^{i \arg\left[ \overline{c_{k,l}^1} c_{k,l}^2 \right]}, & \text{wenn } \dfrac{\left| \overline{c_{k,l}^1} c_{k,l}^2 \right|}{\left| c_{k,l}^1 \right|^2} \geq 1 + \dfrac{\lambda}{2 \left| c_{k,l}^1 \right|^2} \\[4mm] \dfrac{\left| \overline{c_{k,l}^1} c_{k,l}^2 \right| + \dfrac{\lambda}{2}}{\left| c_{k,l}^1 \right|^2} \cdot e^{i \arg\left[ \overline{c_{k,l}^1} c_{k,l}^2 \right]}, & \text{wenn } \dfrac{\left| \overline{c_{k,l}^1} c_{k,l}^2 \right|}{\left| c_{k,l}^1 \right|^2} \leq 1 - \dfrac{\lambda}{2 \left| c_{k,l}^1 \right|^2} \\[4mm] \equiv 1, & \text{sonst} \end{cases}$$

Zur Detektion von Peaks in verrauschten MALDI-Signalen wird jetzt folgendes Schema verwendet.

Das ursprüngliche Signal $f$ wird in kleine Teile $f_n$ unterteilt, wobei aufeinanderfolgende Signalteile $f_n$ und $f_{n+1}$ eine gewisse Überlappung (z.B. 50%) haben.

Jetzt wird die zugehörige Maske $m_n$ nach obiger Gleichung und gewünschtem Regularisierungsterm berechnet, sodass $f_n$ in $f_{n+1}$ transformiert wird.

Sind sich beide Teile $f_n$ und $f_{n+1}$ ähnlich, so ist demnach der Regularisierungsterm nahezu Null.

Ist allerdings in $f_{n+1}$ ein Peak präsent, der nicht in $f_n$ vorkommt (oder umgekehrt), so ist $d(m)$ von Null verschieden und repräsentiert somit einen Peak.

Das zusammengefasste Verfahren dieser Idee lautet wie folgt beschrieben:

Eingabe: Signal $f$,

Länge der Schnitte $L$,

enger Frame $G(g, x_0, \omega_0)$ mit Länge $L$,

Überlappung $q$,

Regulaisierungsparameter $\lambda$

berechne die Zahl $N$ von allen Schnitten von $f$ mit der Überlappung $q$

Für alle Schnitte $f_n$, mit $n \in [1, N-1]$ mache Folgendes

berechne $V_g f_n$ and $V_g f_{n+1}$ with $G(g, x_0, \omega_0)$

nähere $m$ für $V_g f_n$ and $V_g f_{n+1}$ mit $\lambda$

wenn $d(m_n) = \| |m_n| - \mathbb{1} \|_1$ nicht gleich Null ist

berechne die genaue Peakposition von $m_n$

Ende

Ende

**Patentansprüche**

1. Verfahren zur Analyse eines Datensatzes einer Flugzeit-Massenspektrometrie-Messung zur Detektion von Intensitätsmaxima in Form von Peaks, wobei eine Flugzeit von beschleunigten Massen mit Ladungen gemessen wird, insbesondere eines Datensatzes umfassend eine Intensität der Ionenhäufigkeit bezogen auf eine Masse pro Ladung, insbesondere von biologischen Molekülen, insbesondere gewonnen aus einem MALDI-Verfahren, Matrix-assisted laser desorption/ionization, wobei Peakinformation von Rauschen getrennt wird,
wobei mittels eines Transformationsmittels eine 2-Parameter-Transformation aus dem Datensatz der Flugzeit-Massenspektrometrie-Messung in einen Zeit-Frequenz-Phasenraum vorgenommen wird, wobei der Zeit-Frequenz-Phasenraum der Raum ist, der von den zwei Parametern (k, l) der 2-Parameter-Transformation aufgespannt wird, wobei mit dem Transformationsmittel eine Wavelet- Transformation oder eine Gabor Transformation vorgenommen wird,
wobei mit einem Datensatzaufteilungsmittel das Signal des Datensatzes in einzelne, zu vergleichende Signalabschnitte $f_n$ unterteilt wird, wobei aufeinanderfolgende Signalabschnitte $f_n$ und $f_{n+1}$ insbesondere eine vorbestimmte Überlappung in einem Fenster aufweisen, insbesondere etwa 25%, 30% , 35% oder 50 % einer vorbestimmten Fensterbreite,
wobei durch das Transformationsmittel ein Zusammenhang des Signals $f_n$ zu dem zweiten, benachbarten Signal $f_{n+1}$ hergestellt wird dadurch, dass zur Kennzeichnung der Ähnlichkeit der zwei Signale $f_n$ und $f_{n+1}$ ein Multiplier

$G_m : L^2(\mathbb{R}) \to L^2(\mathbb{R})$, der Wavelet-Multiplier oder der Gabor-Multiplier, bestimmt wird, der das erste Signal $f_n$ in das zweite $f_{n+1}$ überführt,

$$f_{n+1} = G_m f_n,$$

insbesondere in einer Näherung

$$f_{n+1} = G_m f_n \qquad = V_g^*(m \cdot V_g f_n)$$

wobei

$V_g : L^2(\mathbb{R}) \to \ell^2(\mathbb{Z}^2)$ = Analysisoperator der 2-Parameter-Transformation, mit einem Fensterfunktionsmittel g(t),

$V_g^*$ = zugehöriger adjungierter Operator der 2-Parameter-Transformation,

$G_m : L^2(\mathbb{R}) \to L^2(\mathbb{R})$ Gabor Multiplier oder Wavelet-Multiplier, der die 2-Parameter-Transformation zwischen dem ersten Signals $f_n$ zu einem zweiten Signal $f_{n+1}$ mit unterschiedlichen Phasenraumcharakteristika, insbesondere Zeit-Frequenz(modulieren) Charakteristika durch multiplikative Modifikation beschreibt, wobei

$m \in \ell^\infty(\mathbb{Z}^2)$ = Maske des jeweiligen Wavelet oder Gabor Multipliers,

wobei durch Anpassung einer Maske m eine Näherung durch Multiplikation im Bereich Zeit/Frequenz vorgenommen wird,
wobei die Maske $m$ nahezu 1, entspricht, wenn die Signale $f_n$ und $f_{n+1}$ sehr ähnlich sind,
wobei die Maske $m$ von 1 verschieden ist, wenn in $f_n$ und/oder $f_{n+1}$ ein Peak existiert, der nicht in dem anderen Bereich $f_n$ und/oder $f_{n+1}$ vorkommt.

2. Verfahren nach Anspruch 1, **dadurch gekennzeichnet, dass** für die Fensterfunktion g(t) gilt:
für die Gabor-Transformation $V_g$ angewandt auf ein Signal $f(t)$:

$$V_g f(x,\omega) = \int_{\mathbb{R}} f(t)\, \bar{g}(t-x) e^{-2\pi i t\omega}\, dt$$

und für die Wavelet-Transformation $V_g$ angewandt auf ein Signal $f(t)$:

$$V_g f(x,a) = a^{-1/2} \int_{\mathbb{R}} f(t)\, \bar{g}(a^{-1} \cdot (t-x))\, dt$$

3. Verfahren nach Anspruch 1 oder 2, **dadurch gekennzeichnet, dass** der Multiplier $G_m$, der Wavelet-Multiplier oder der Gabor-Multiplier, in einer linearen Näherung nach der folgenden Gleichung bestimmt wird:

$$m = \arg\min_m \| f_{n+1} - G_m f_n \|^2 + \lambda d(m)$$
$$= \arg\min_m \| V_g^*(V_g f_{n+1} - m \cdot V_g f_n) \|^2 + \lambda d(m)$$

wobei

$\lambda$ = Regularisierungsparameter,
$d(m)$ = ein Regularisierungsterm ist, wobei der Regularisierungsterm so gewählt wird, dass er möglichst klein ist, wenn sich die beiden Signale $f_n$ und $f_{n+1}$ ähnlich sind, insbesondere in beiden Signalen ähnliche Peaks vorhanden sind.

4. Verfahren nach Anspruch 3, **dadurch gekennzeichnet, dass** als Regularisierungsterm $d(m) = \||m| - 1\|_1$ verwendet wird, wobei eine Abweichung vom absoluten Wert 1 der Maske $m$ erzwungen wird und/oder $d(m) = \|m - 1\|^2$ zur Kontrolle der Energie der Maske verwendet wird.

5. Verfahren nach Anspruch 3 bis 4, **dadurch gekennzeichnet, dass** eine diagonale Näherung verwendet wird, wobei im Zeit-Frequenz Phasenraum angesetzt wird:

$$m = \arg\min_{m} \sum \sum_{k,l \in \mathbb{Z}} \left| c_{k,l}^{n+1} - m_{k,l} c_{k,l}^{n} \right|^{2} + \lambda d(m).$$

wobei das Signal $f \in L^{2}(\mathbb{R})$ in diskreter Zerlegung im Zeit-Frequenz Phasenraum dargestellt wird

$$f = V_{g}^{*} V_{g} f = \sum \sum_{k,l \in \mathbb{Z}} c_{k,l} g_{k,l} = \sum \sum_{k,l \in \mathbb{Z}} \langle f, g_{k,l} \rangle g_{k,l}$$

mit $c_{k,\ell} = \langle f, g_{k,\ell} \rangle$ = Zeit-Frequenz Koeffizienten von $f_{n}$ im Phasenraum (k,l).
wobei insbesondere $g_{k,l} = e^{2\pi i l \omega_{0}} g(t - kx_{0})$ = modulierte, translatierte Fensterfunktionen g im Rahmen der Gabor-Transformation,

wobei insbesondere $g_{k,l} = 2^{-\frac{l}{2}} g(2^{-l}(t - kx_{0}))$ modulierte, translatierte Fensterfunktionen g im Rahmen der Wavelet-Transformation

wobei in Abhängigkeit vom Regulierungsterm d(m) und/oder dem Regulierungsparameter $\lambda$ gelöst wird:

    2.

$$d(m) = \|m - 1\|^{2}$$

$$m_{k,l} = \frac{\left| \overline{c_{k,l}^{n}} c_{k,l}^{n+1} \right| + \lambda}{\left| c_{k,l}^{n} \right|^{2} + \lambda} \cdot e^{i \arg\left[ \overline{c_{k,l}^{n}} c_{k,l}^{n+1} \right]}$$

    3.

$$d(m) = \||m| - 1\|_{1}$$

$$m_{k,l} = \begin{cases} \dfrac{\left| \overline{c_{k,l}^{n}} c_{k,l}^{n+1} \right| - \dfrac{\lambda}{2}}{\left| c_{k,l}^{n} \right|^{2}} \cdot e^{i \arg\left[ \overline{c_{k,l}^{n}} c_{k,l}^{n+1} \right]}, & \text{wenn } \dfrac{\left| \overline{c_{k,l}^{n}} c_{k,l}^{n+1} \right|}{\left| c_{k,l}^{n} \right|^{2}} \geq 1 + \dfrac{\lambda}{2\left| c_{k,l}^{n} \right|^{2}} \\[2em] \dfrac{\left| \overline{c_{k,l}^{n}} c_{k,l}^{n+1} \right| + \dfrac{\lambda}{2}}{\left| c_{k,l}^{n} \right|^{2}} \cdot e^{i \arg\left[ \overline{c_{k,l}^{n}} c_{k,l}^{n+1} \right]}, & \text{wenn } \dfrac{\left| \overline{c_{k,l}^{n}} c_{k,l}^{n+1} \right|}{\left| c_{k,l}^{n} \right|^{2}} \leq 1 - \dfrac{\lambda}{2\left| c_{k,l}^{n} \right|^{2}} \\[2em] \equiv 1, & \text{sonst} \end{cases}$$

**6.** Verfahren nach einem der Ansprüche 1 bis 5, **dadurch gekennzeichnet, dass** $m_{k,l}$ die Maske, $k$ der Zeitindex und $l$ der Frequenzindex so wird die exakte Zeitposition $f_{k}$ eines Peaks derart bestimmt, dass für jeden Zeitpunkt die Absolutbeträge der Frequenzen aufsummiert werden: $f_{k} = \Sigma_{l} |m_{k,l}|$.

**7.** Verfahren nach einem der Ansprüche 1 bis 6, **dadurch gekennzeichnet, dass** es in Verbindung mit einem bildgebenden Verfahrens, insbesondere zur Darstellung eines Ausgabebildes mit Peakdarstellungen, eingesetzt wird.

**8.** Verwendung eines Verfahrens nach einem der Ansprüche 1 bis 7, für die Messung von Proteinen und/oder Peptiden und/oder Oligonukleotiden und/oder Oligosachariden verwendet wird.

9. Verwendung eines Verfahrens nach einem der Ansprüche 1 bis 7, zur Messung von Medikamenten und/oder Drogen und/oder Dopingmittel und/oder Gewebeschnitten und/oder Tumorausbreitungsproben.

10. Vorrichtung zur Analyse zur Anwendung eines Verfahrens nach einem der Ansprüche 1 bis 9, wobei vorgesehen ist: ein Mittel zur Erfassung von Maldi-Datensätzen, ein Mittel zum Berechnen eine 2-Parameter-Transformation, Wavelet-Transformation oder Gabor-Transformation, nach einem der Ansprüche 1 bis 9, ein Mittel zur Ausgabe des Berechnungsergebnisses mit einem Ergebnis, ob ein Peak vorliegt oder nicht.

**Claims**

1. Method for the analysis of a data set of a time-of-flight mass spectrometry measurement for the detection of intensity maxima in the form of peaks, wherein a time-of-flight of accelerated masses with charges is measured, in particular of a data set comprising an intensity of the ion frequency related to a mass per charge, in particular of biological molecules, in particular obtained from a MALDI method, matrix-assisted laser desorption/ionization, wherein peak information is separated from noise,
   wherein a 2-parameter transformation from the data set of the time-of-flight mass spectrometry measurement into a time-frequency phase space is carried out by means of a transformation means, wherein the time-frequency phase space is the space which is spanned by the two parameters (k, l) of the 2-parameter transformation,
   a wavelet transformation or a Gabor transformation being carried out with the transformation means, the signal of the data set being subdivided into individual signal sections $f_n$ to be compared with a data set subdivision means,
   successive signal sections $f_n$ and/or $f_{n+1}$ in particular having a predetermined overlap in a window, in particular about 25%, 30%, 35% or 50% of a predetermined window width,
   the transformation means establishing a relationship between the signal $f_n$ and the second, adjacent signal $f_{n+1}$ by determining a multiplier $G_m \colon L^2(\mathbb{R}) \to L^2(\mathbb{R})$, the wavelet multiplier or the Gabor multiplier, for identifying the similarity of the two signals $f_n$ and/or $f_{n+1}$,
   which multiplier converts the first signal $f_n$ into the second signal $f_{n+1}$

$$f_{n+1} = G_m f_n,$$

especially in an approximation

$$f_{n+1} = G_m f_n \qquad = V_g^*(m \cdot V_g f_n)$$

Whereas
   $V_g \colon L^2(\mathbb{R}) \to \ell^2(\mathbb{Z}^2)$ = Analysis operator of the 2-parameter transformation, with a window function means g(t),
   $V_g^*$ = associated adjoint operator of the 2-parameter transformation,
   $G_m \colon L^2(\mathbb{R}) \to L^2(\mathbb{R})$ = Gabor multiplier or wavelet multiplier, which describes the 2-parameter transformation between the first signal $f_n$ and a second signal $f_{n+1}$ with different phase space characteristics, in particular time-frequency (modulate) characteristics, by multiplicative modification, where
   $m \in \ell^\infty(\mathbb{Z}^2)$ = Mask of the respective multiplier, Wavelet or Gabor Multipliers,

wherein an approximation by multiplication in the time/frequency range is carried out by adapting a mask m, where the mask m is nearly 1, if the signals $f_n$ and $f_{n+1}$ are very similar,
where the mask m is different from 1 if a peak exists $f_n$ and/or $i_{n+1}$ which does not occur in the other region $f_n$ and/or $f_{n+1}$.

2. Method according to claim 1, **characterized in that** the window function g(t) applies:
   for the Gabor transformation $V_g$ applied to a signal $f(t)$:

$$V_g f\,(x,\omega) = \int_{\mathbb{R}} f(t)\,\bar{g}(t-x)e^{-2\pi it\omega}dt$$

and for the wavelet transformation $V_g$, applied to a signal f(t):

$$V_g f\,(x,a) = a^{-1/2}\int_{\mathbb{R}} f(t)\,\bar{g}(a^{-1}\cdot(t-x))dt$$

3. Method according to claim 1 or 2, **characterized in that** the multiplier $G_{m'}$, in particular the wavelet multiplier or the Gabor multiplier, is determined in a linear approximation according to the following equation:.

$$m = \arg\min_m \| f_{n+1} - G_m f_n \|^2 + \lambda d(m)$$
$$= \arg\min_m \left\| V_g^*(V_g f_{n+1} - m\cdot V_g f_n)\right\|^2 + \lambda d(m)$$

where

A = regularization parameter,
d(m) = a regularization term, the regularization term being selected such that it is as small as possible if the two signals $f_n$ et $f_{n+1}$ are similar, in particular if similar peaks are present in both signals.

4. Method according to claim 3, **characterized in that** $d(m) = ||m| - 1||_1$ is used as regularization term, a deviation from the absolute value 1 of the mask m being forced and/or $d(m) = ||m - 1||^2$ being used to control the energy of the mask.

5. Method according to claims 3 to 4, **characterized in that** a diagonal approximation is used, phase space being applied in time-frequency:

$$m = \arg\min_m \sum \sum_{k,l\in\mathbb{Z}} \left| c_{k,l}^{n+1} - m_{k,l}c_{k,l}^n \right|^2 + \lambda d(m).$$

where the signal $f \in L^2(\mathbb{R})$ is represented in discrete decomposition in time-frequency phase space

$$f = V_g^* V_g f = \sum \sum_{k,l\in\mathbb{Z}} c_{k,l}g_{k,l} = \sum \sum_{k,l\in\mathbb{Z}} \langle f, g_{k,l}\rangle g_{k,l}$$

with $c_{k,l} = \langle f, g_{k,l}\rangle$ = time-frequency coefficients of $f_n$ in phase space (k,l),
where in particular $g_{k,l} = e^{2\pi i l\omega_0 t}g(t - kx_0)$ = modulated, translated window functions g in the context of the Gabor transformation,

where, in particular, $g_{k,l} = 2^{-\frac{l}{2}}g(2^{-l}(t - kx_0))$ modulated, translated window functions g in the context of the wavelet transformation

where d(m) and/or the regulation parameter A is solved as a function of the regulation term:

1.

$$d(m) = \|m - 1\|^2$$

$$m_{k,l} = \frac{\left|\overline{c_{k,l}^{n}}\,c_{k,l}^{n+1}\right| + \lambda}{\left|c_{k,l}^{n}\right|^{2} + \lambda} \cdot e^{\,i\,\arg\left[\overline{c_{k,l}^{n}}\,c_{k,l}^{n+1}\right]}$$

2.

$$d(m) = \big\|\,|m| - 1\,\big\|_{1}$$

$$m_{k,l} = \begin{cases} \dfrac{\left|\overline{c_{k,l}^{n}}\,c_{k,l}^{n+1}\right| - \dfrac{\lambda}{2}}{\left|c_{k,l}^{n}\right|^{2}} \cdot e^{\,i\,\arg\left[\overline{c_{k,l}^{n}}\,c_{k,l}^{n+1}\right]}, & \text{if } \dfrac{\left|\overline{c_{k,l}^{n}}\,c_{k,l}^{n+1}\right|}{\left|c_{k,l}^{n}\right|^{2}} \ge 1 + \dfrac{\lambda}{2\left|c_{k,l}^{n}\right|^{2}} \\[2em] \dfrac{\left|\overline{c_{k,l}^{n}}\,c_{k,l}^{n+1}\right| + \dfrac{\lambda}{2}}{\left|c_{k,l}^{n}\right|^{2}} \cdot e^{\,i\,\arg\left[\overline{c_{k,l}^{n}}\,c_{k,l}^{n+1}\right]}, & \text{if } \dfrac{\left|\overline{c_{k,l}^{n}}\,c_{k,l}^{n+1}\right|}{\left|c_{k,l}^{n}\right|^{2}} \le 1 - \dfrac{\lambda}{2\left|c_{k,l}^{n}\right|^{2}} \\[2em] \equiv 1, & \text{else} \end{cases}$$

6.  Method according to one of the claims 1 to 5, **characterized in that** $m_{k,l}$ the mask, $k$ the time index and $l$ the frequency index the exact time position $f_k$ of a peak is determined in such a way that for each instant the absolute amounts of the frequencies are summed up: $f_k = \sum_l |m_{k,l}|$.

7.  Method according to one of claims 1 to 6, **characterized in that** it is used in conjunction with an imaging method, in particular for displaying an output image with peak representations.

8.  Use of a method according to any of Claims 1 to 7 for the measurement of proteins and/or peptides and/or oligonucleotides and/or oligosaccharides.

9.  Use of a method according to any of Claims 1 to 7 for the measurement of drugs and/or drugs and/or doping agents and/or tissue sections and/or tumour spread samples.

10. Analysis apparatus for applying a method according to one of claims 1 to 9, comprising: means for acquiring Maldi data sets; means for calculating a 2-parameter transformation, in particular a wavelet transformation or a Gabor transformation, according to one of claims 1 to 9; means for outputting the calculation result with a result as to whether a peak is present or not.

**Revendications**

1.  Procédé d'analyse d'un ensemble de données d'une mesure par spectrométrie de masse à temps de vol pour la détection de maxima d'intensité sous forme de pics, dans lequel on mesure un temps de vol de masses accélérées avec charges, en particulier d'un ensemble de données comprenant une intensité de la fréquence ionique liée à une masse par charge, en particulier de molécules biologiques, en particulier obtenues par un procédé MALDI, de désorption/ionisation laser assisté par matrice, où les informations des pics sont séparées du bruit,
    dans laquelle, au moyen d'un moyen de transformation, une transformation à deux paramètres est effectuée à partir de l'ensemble de données de la mesure de spectrométrie de masse dans le temps de vol en un espace de phase temps-fréquence, l'espace de phase temps-fréquence étant l'espace qui est couvert par les deux paramètres (k, l) de la transformation à 2 paramètres,
    dans laquelle une transformation par ondelettes ou une transformation de Gabor est effectuée avec les moyens de transformation, le signal de l'ensemble de données étant subdivisé en sections de signal individuelles $f_n$ à comparer à un moyen de subdivision d'ensemble de données, les sections de signal successives $f_n$ et $f_{n+1}$ en particulier ayant un recouvrement prédéterminé dans une fenêtre, en particulier environ 25%, 30%, 35% ou 50% d'une largeur prédéterminée,
    la transformation signifie établir une relation entre le signal $f_n$ et le second signal adjacent $f_{n+1}$ en déterminant un multiplicateur $G_m: L^2(\mathbb{R}) \to L^2(\mathbb{R})$, le multiplicateur d'ondelettes ou le multiplicateur de Gabor, qui convertit le premier signal $f_n$ en le second signal $f_{n+1}$, afin de déterminer la similarité des deux signaux $f_n$ et/ou $f_{n+1}$,

$$f_{n+1} = G_m f_n,$$

en particulier dans une approximation

$$f_{n+1} = G_m f_n \qquad = V_g^*(m \cdot V_g f_n)$$

là où

$V_g: L^2(\mathbb{R}) \to \ell^2(\mathbb{Z}^2)$ = Opérateur d'analyse de la transformation à 2 paramètres, avec une fonction fenêtre signifie g(t),

$V_g^*$ = Opérateur adjoint associé de la transformation à 2 paramètres,

$G_m: L^2(\mathbb{R}) \to L^2(\mathbb{R})$ = multiplicateur de Gabor ou multiplicateur d'ondelettes, qui décrit la transformation à 2 paramètres entre le premier signal $f_n$ et un second signal $f_{n+1}$ avec différentes caractéristiques d'espace de phase, en particulier caractéristiques temps-fréquence (modulation) par modification multiplicative, où

$m \in \ell^\infty(\mathbb{Z}^2)$ = Masque du multiplicateur respectif, des multiplicateurs d'ondelettes ou de Gabor,

dans laquelle une approximation par multiplication dans la gamme temps/fréquence est effectuée en adaptant un masque m,
où le masque m est presque 1, si les signaux $f_n$ et $f_{n+1}$ sont très similaires,
où le masque m est différent de 1 si un pic existe dans $f_n$ et/ou $f_{n+1}$ qui ne se produit pas dans l'autre région $f_n$ et/ou $f_{n+1}$.

2. Procédé selon la revendication 1, **caractérisé en ce que** la fonction de fenêtre g(t) s'applique :
pour la transformation de Gabor $V_g$ appliquée à un signal $f(t)$:

$$V_g f(x, \omega) = \int_{\mathbb{R}} f(t)\, \bar{g}(t - x)e^{-2\pi i t \omega} dt$$

et pour la transformation en ondelettes $V_g$ appliquée à un signal $f(t)$:

$$V_g f(x, a) = a^{-1/2} \int_{\mathbb{R}} f(t)\, \bar{g}(a^{-1} \cdot (t - x)) dt$$

3. Procédé selon la revendication 1 ou 2, **caractérisé en ce que** le multiplicateur $G_m$, en particulier le multiplicateur d'ondelettes ou le multiplicateur de Gabor, est déterminé selon une approximation linéaire selon l'équation suivante :

$$m = \arg \min_m \| f_{n+1} - G_m f_n \|^2 + \lambda d(m)$$
$$= \arg \min_m \| V_g^*(V_g f_{n+1} - m \cdot V_g f_n) \|^2 + \lambda d(m)$$

là où

$\lambda$ = paramètre de régularisation,
$d(m)$ = un terme de régularisation, le terme de régularisation étant sélectionné de telle sorte qu'il soit le plus petit possible si les deux signaux $f_n$ et $f_{n+1}$ sont similaires, en particulier si des pics similaires sont présents dans les deux signaux.

4. Procédé selon la revendication 3, **caractérisé en ce que** $d(m) = \||m| - 1\|_1$ est utilisé comme terme de régularisation, un écart par rapport à la valeur absolue 1 du masque m étant forcé et/ou $d(m) = \|m - 1\|^2$ étant utilisé pour contrôler l'énergie du masque.

5. Procédé selon les revendications 3 à 4, **caractérisé en ce qu'**on utilise une approximation diagonale, l'espace de phase étant appliqué en temps-fréquence :

$$m = \arg\min_{m} \sum \sum_{k,l \in \mathbb{Z}} \left| c_{k,l}^{n+1} - m_{k,l} c_{k,l}^{n} \right|^2 + \lambda d(m).$$

où le signal $f \in L^2(\mathbb{R})$ est représenté en décomposition discrète dans l'espace de phase temps-fréquence.

$$f = V_g^* V_g f = \sum \sum_{k,l \in \mathbb{Z}} c_{k,l} g_{k,l} = \sum \sum_{k,l \in \mathbb{Z}} \langle f, g_{k,l} \rangle g_{k,l}$$

avec $c_{k,l} = \langle f, g_{k,l} \rangle$ = coefficients temps-fréquence de $f_n$ en espace de phase (k,l).
où en particulier $g_{k,l} = e^{2\pi i l \omega_0 t} g(t - kx_0)$ = fonctions de fenêtre modulées et traduites g dans le contexte de la transformation de Gabor,

où, en particulier, $g_{k,l} = 2^{-\frac{l}{2}} g(2^{-l}(t - kx_0))$ modulé, les fonctions de fenêtre traduites g dans le contexte de la transformation en ondelettes

où d(m) et/ou le paramètre de régulation $\lambda$ est résolu en fonction du terme de régulation :

1.

$$d(m) = \|m - 1\|^2$$

$$m_{k,l} = \frac{\left| \overline{c_{k,l}^{n}} c_{k,l}^{n+1} \right| + \lambda}{\left| c_{k,l}^{n} \right|^2 + \lambda} \cdot e^{i \arg\left[ \overline{c_{k,l}^{n}} c_{k,l}^{n+1} \right]}$$

2.

$$d(m) = \|\,|m| - 1\,\|_1$$

$$m_{k,l} = \begin{cases} \dfrac{\left| \overline{c_{k,l}^{n}} c_{k,l}^{n+1} \right| - \dfrac{\lambda}{2}}{\left| c_{k,l}^{n} \right|^2} \cdot e^{i \arg\left[ \overline{c_{k,l}^{n}} c_{k,l}^{n+1} \right]}, & \text{quand } \dfrac{\left| \overline{c_{k,l}^{n}} c_{k,l}^{n+1} \right|}{\left| c_{k,l}^{n} \right|^2} \geq 1 + \dfrac{\lambda}{2 \left| c_{k,l}^{n} \right|^2} \\[2em] \dfrac{\left| \overline{c_{k,l}^{n}} c_{k,l}^{n+1} \right| + \dfrac{\lambda}{2}}{\left| c_{k,l}^{n} \right|^2} \cdot e^{i \arg\left[ \overline{c_{k,l}^{n}} c_{k,l}^{n+1} \right]}, & \text{quand } \dfrac{\left| \overline{c_{k,l}^{n}} c_{k,l}^{n+1} \right|}{\left| c_{k,l}^{n} \right|^2} \leq 1 - \dfrac{\lambda}{2 \left| c_{k,l}^{n} \right|^2} \\[2em] \equiv 1, & \text{à d'autres égards} \end{cases}$$

6. Procédé selon l'une des revendications 1 à 5, **caractérisé en ce que** $m_{k,l}$ le masque, $k$ l'indice de temps et $l$ l'indice de fréquence la position temporelle exacte $f_k$ d'un pic est déterminée de telle manière que pour chaque instant les quantités absolues des fréquences sont additionnées : $f_k = \sum_l |m_{k,l}|$.

**7.** Procédé selon l'une des revendications 1 à 6, **caractérisé en ce qu'**il est utilisé conjointement avec un procédé d'imagerie, en particulier pour l'affichage d'une image de sortie avec représentation des pics.

**8.** Utilisation d'un procédé selon l'une quelconque des revendications 1 à 7 pour la mesure de protéines et/ou de peptides et/ou d'oligonucléotides et/ou d'oligosaccharides.

**9.** l'utilisation d'un procédé selon l'une quelconque des revendications 1 à 7 pour la mesure de médicaments et/ou de drogues et/ou d'agents dopants et/ou de sections de tissus et/ou d'échantillons de propagation de tumeurs.

**10.** Appareil d'analyse pour appliquer un procédé selon l'une quelconque des revendications 1 à 9, comprenant : un moyen pour acquérir des ensembles de données de Maldi ; un moyen pour calculer une transformation à 2 paramètres, en particulier une transformation en ondelettes ou une transformation de Gabor, selon l'une quelconque des revendications 1 à 9 ; un moyen pour sortir le résultat du calcul avec un résultat déterminant si un pic est présent ou non.

Fig. 1

**IN DER BESCHREIBUNG AUFGEFÜHRTE DOKUMENTE**

**In der Beschreibung aufgeführte Nicht-Patentliteratur**

- **DU et al.** *Bioinformatics,* 2006, vol. 22 (17), 2059-2065